# EUROPÄISCHE PATENTANMELDUNG

(11) **EP 4 796 068 A1**
(43) Veröffentlichungstag der Anmeldung: **26.08.2026**
(21) Anmeldenummer: 26153277.4
(22) Anmeldetag: 21.01.2026
(51) Int. Cl.: A61B 50/34, A61B 50/20

(54) **VORRICHTUNG ZUM LAGERN VON INSTRUMENTEN ODER IMPLANTATEN AUF EINEM INSTRUMENTENSIEB**

(30) Priorität: 20.02.2025 DE 102025106555
(71) Anmelder: Aesculap AG, 78532 Tuttlingen (DE)
(72) Erfinder: Rosin, Bianca, 78576 Emmingen-Liptingen (DE)
(74) Vertreter: DREISS Patentanwälte PartG mbB

(57) **Zusammenfassung**

Die Erfindung betrifft eine Vorrichtung (10) zum Lagern eines oder mehrerer ärztlicher, insbesondere chirurgischer, Instrumente und/oder Implantate auf einem Instrumentensieb (2) zur Verwendung bei der Sterilgutversorgung im medizinischen Bereich mit einer Siebträgerstruktur (4) mit Sieböffnungen (6), wobei die Vorrichtung (10) ein Instrumentenlagerteil (12) mit einem gegen die Siebträgerstruktur (4) anlegbaren Basisabschnitt (14) und mit wenigstens einem sich von dem Basisabschnitt (14) emporerhebenden Wandabschnitt (16) für die Aufnahme der Instrumente und/oder Implantate und ein Befestigungselement (18) zum lösbaren Festlegen des Instrumentenlagerteils (12) an der Siebträgerstruktur (4) umfasst; **erfindungsgemäß wird vorgeschlagen, dass** das Befestigungselement (18) wenigstens eine in Richtung auf die Siebträgerstruktur (4) des Instrumentensiebs (2) sich erstreckende und damit verrastbare Federzunge (28) und wenigstens einen gegen den Basisabschnitt (14) des Instrumentenlagerteils (12) abstützbaren Stützschenkel (32) aufweist, und dass in dem Basisabschnitt (14) des Instrumentenlagerteils (12) eine auf die Außenkontur des Befestigungselements (18) derart abgestimmte und durch den Basisabschnitt (14) hindurchgehende Ausnehmung (20) ausgebildet ist, dass das Befestigungselement (18) mitsamt seiner Federzunge (28) in die Ausnehmung (20) eintauchbar ist, so dass die Federzunge (28) des Befestigungselements (18) mit der Siebträgerstruktur (4) verrastbar ist und sich das Befestigungselement (18) dann mit seinem Stützschenkel (32) gegen eine Seite (34) des Basisabschnitts (14) des Instrumentenlagerteils (12) abstützt und hierdurch an dem Instrumentensieb (2) fixiert ist.

## Beschreibung

Die Erfindung betrifft eine Vorrichtung zum Lagern und vorzugsweise zum Halten eines oder mehrerer ärztlicher, insbesondere chirurgischer, Instrumente und/oder Implantate in einer bestimmungsgemäßen Anordnung auf einem Instrumentensieb zur Verwendung bei der Sterilgutversorgung im medizinischen Bereich mit einer Siebträgerstruktur mit einer Vielzahl von Sieböffnungen, wobei die Vorrichtung ein, insbesondere metallisches Instrumentenlagerteil mit einem gegen die Siebträgerstruktur anlegbaren Basisabschnitt und mit wenigstens einem sich von dem Basisabschnitt emporerhebenden Wandabschnitt für die Aufnahme der Instrumente und/oder Implantate und ein Befestigungselement zum lösbaren Festlegen des Instrumentenlagerteils an der Siebträgerstruktur des Instrumentensiebs umfasst. Ferner betrifft die Erfindung ein Instrumentensieb zur Verwendung bei der Sterilgutversorgung im medizinischen Bereich.

Instrumentensiebe, die häufig als Instrumentensiebkörbe oder Siebeinsätze für Sterilgutcontainer ausgebildet werden, sowie eingangs genannte Vorrichtungen zum Lagern und Halten von Instrumenten und/oder Implantaten bei solchen Instrumentensieben spielen eine wichtige Rolle im sogenannten Sterilgutkreislauf ärztlicher und insbesondere chirurgischer Instrumente und teilweise auch Implantaten. Sie nehmen die zu sterilisierenden Instrumente während der Sterilisation und auch danach im Zuge der Verbringung in das Sterilgutlager und von dort zum Ort der Verwendung, insbesondere einen Operationsraum, auf. Hierfür sind bereits zahlreiche sogenannte Organisationssysteme für Instrumentensiebe vorgeschlagen worden, um die Instrumente in einer vorbestimmten Lage und Anordnung auf den Instrumentensieben vorzuhalten, bis sie unmittelbar vor ihrer Verwendung von medizinischem Personal entnommen werden. Vorbekannte Vorrichtungen zum Lagern eines oder mehrerer Instrumente auf dem Instrumentensieb sind entweder mittels Schraubverbindung an dem Instrumentensieb bzw. dessen Siebträgerstruktur befestigbar, was als aufwändig angesehen wird, oder sie werden von der Unterseite der Siebträgerstruktur her durch Sieböffnungen oder sonstige speziell ausgebildete Öffnungen durch die Siebträgerstruktur hindurchgesteckt, so dass sie auf der Oberseite des Instrumentensiebs nach oben vorstehen, um dort Instrumente lagern zu können. Beides impliziert eine Montage der Haltevorrichtung gewissermaßen von beiden Seiten des Instrumentensiebs. Die Lager- oder Haltevorrichtungen können demgemäß nur vor der eigentlichen Beladung mit Instrumenten auf der Siebträgerstruktur konfiguriert werden, da das Instrumentensieb hierfür verkippt und gewendet werden muss. Auch erweisen sich an der Unterseite des Instrumentensiebs angeordnete Montagemittel als störend; sie können insbesondere zu Abrieb, Beschädigung der Stellfläche oder bei ungleichmäßiger Verteilung zu einem Wackeln des Instrumentensiebs auf der Stellfläche führen.

Beispielsweise existieren metallische Instrumentenlagerteile, die aus einem Blechstreifenabschnitt durch wenige Biege-/Stanzschnitte gefertigt sind. Sie haben einen im Wesentlichen ebenen streifenförmigen Basisabschnitt und von dessen Enden sich emporhebende Wandabschnitte, welche insbesondere durch V-förmige Formgebung oder auch in sonstiger Weise Auflagestellen für die Aufnahme eines Instruments oder Implantats aufweisen. Diese Instrumentenlagerteile werden typischerweise mit Schrauben und Muttern an der Siebträgerstruktur eines Instrumentensiebs festgeschraubt, was als aufwändig anzusehen ist. Es wird zum einen ein Hilfswerkzeug für die Montage benötigt. Zum anderen muss der Zugang zum Instrumentensieb von oben und unten erfolgen. Man muss eine Schraube von unten durch eine Sieböffnung hindurchführen und festhalten und ferner den streifenförmigen Basisabschnitt des Instrumentenlagerteils gegen Verdrehen festhalten und gleichzeitig eine Mutter mit einem Werkzeug festziehen.

Hiervon ausgehend liegt der vorliegenden Erfindung die Aufgabe zugrunde, die lösbare Anordnung einer Vorrichtung der eingangsgenannten Art zum Lagern eines oder mehrerer Instrumente oder Implantate auf einem Instumentensieb bedienungsfreundlicher zu gestalten, wobei das lösbare Befestigen der Vorrichtung an der Siebträgerstruktur werkzeuglos ausgeführt werden können soll.

Diese Aufgabe wird durch eine Vorrichtung der eingangs genannten Art erfindungsgemäß dadurch gelöst, dass das Befestigungselement wenigstens eine in Richtung auf die Siebträgerstruktur des Instrumentensiebs sich erstreckende und damit verrastbare Federzunge und wenigstens einen gegen den Basisabschnitt des Instrumentenlagerteils abstützbaren Stützschenkel aufweist, und dass in dem Basisabschnitt des Instrumentenlagerteils eine auf die Außenkontur des Befestigungselements derart abgestimmte und durch den Basisabschnitt hindurchgehende Ausnehmung ausgebildet ist, dass das Befestigungselement mitsamt seiner Federzunge in die Ausnehmung eintauchbar ist, so dass die Federzunge des Befestigungselements mit der Siebträgerstruktur verrastbar ist und sich das Befestigungselement dann mit seinem Stützschenkel gegen eine von der Siebträgerstruktur abgewandte Seite des Basisabschnitts des Instrumentenlagerteils abstützt, so dass das Instrumentenlagerteil hierdurch an dem Instrumentensieb fixiert ist.

Es wird also erfindungsgemäß vorgeschlagen, dass das Befestigungselement für das eigentliche Instrumentenlagerteil selbst mittels wenigstens einer Federzunge mit der Siebträgerstruktur eines Instrumentensiebs verrastbar und auf diese Weise mitsamt dem eigentlichen Instrumentenlagerteil an der Siebträgerstruktur fixierbar ist. Hierfür wird in dem Basisabschnitt des Instrumentenlagerteils die genannte Ausnehmung vorgesehen, in welche das Befestigungselement mitsamt seiner Federzunge einsetzbar ist, so dass die Federzunge durch diese Ausnehmung hindurch in die darunter befindliche Siebträgerstruktur eingreifen kann. Dabei ist also der Basisabschnitt des Instrumentenlagerteils zwischen der Siebträgerstruktur und dem Befestigungselement angeordnet, wobei sich das Befestigungselement mit seinem wenigstens einen Stützschenkel gegen den Basisabschnitt abstützt und hierdurch eine Klemmkraft auf den Basisabschnitt ausübt. Hierdurch wird das Instrumentenlagerteil spielfrei an der Siebträgerstruktur des Instrumentensiebs gehalten. Es wird hierfür als bevorzugt angesehen, wenn das Befestigungselement zwei einander gegenüberliegende Stützschenkel aufweist, da hierdurch über zwei Stellen ein gleichmäßiger Klemmdruck auf den Basisabschnitt des Instrumentenlagerteils ausgeübt werden kann. Es ist weiter denkbar und vorteilhaft, wenn bei dem Basisabschnitt des Instrumentenlagerteils mehrere Ausnehmungen für entsprechend mehrere Befestigungselemente für das eine Instrumentenlagerteil vorgesehen sind. Hierdurch kann auch ein langgestrecktes ausladendes Instrumentenlagerteil sicher an dem Instrumentensieb lösbar fixiert werden. Die lösbare Festlegung des Befestigungselements selbst an der Siebträgerstruktur mittels seiner Federzunge kann wie in DE 10 2023 103 493 A1 bereits beschrieben erfolgen. Die diesbezügliche Offenbarung dieser Publikation wird in die vorliegende Anmeldung einbezogen.

Es erweist sich weiter als besonders vorteilhaft, wenn der Stützschenkel des Befestigungselements federnd ausgebildet ist, so dass verschiedene Dicken des Basisabschnitts des Instrumentenlagerteils hierdurch spielfrei akkommodiert werden können (Anspruch 2). Auf diese Weise kann ein und dasselbe Befestigungselement für verschiedene Instrumentenlagerteile mit unterschiedlich dicken Basisabschnitten verwendet werden.

Weiter wird vorgeschlagen, dass der Ausnehmung in dem Basisabschnitt des Instrumentenlagerteils eine weitere insbesondere schlitzförmige Ausnehmung oder Vertiefung zugeordnet und in dem Basisabschnitt ausgebildet ist, in welche der Stützschenkel des Befestigungselements mit seinem freien Ende eingreifen kann. (Anspruch 3) Dadurch, dass der Stützschenkel in diese weitere insbesondere schlitzförmige Ausnehmung oder Vertiefung in dem Basisabschnitt des Instrumentenlagerteils eingreifen kann, wird die bestimmungsgemäße Anordnung und Montage von Befestigungselement und Instrumentenlagerteil an der Siebträgerstruktur unterstützt und ein unbeabsichtigtes Verdrehen des Befestigungselements relativ zu dem Basisabschnitt verhindert. Bevorzugtermaßen sind jeder Ausnehmung zwei solche schlitzförmige Ausnehmungen oder Vertiefungen auf einander gegenüberliegenden Seiten zur Aufnahme von beidseitigen Stüzschenkeln des Befestigungselements zugeordnet sind.

Es erweist sich weiter als vorteilhaft, wenn sich der Stützschenkel ausgehend von einem in die Ausnehmung in dem Basisabschnitt des Instrumentenlagerteils eintauchbaren dem Instrumentensieb zugewandten unteren Bereich des Befestigungselements durch diese Ausnehmung schräg nach oben über den Basisabschnitt hinaus erstreckt und dann wieder in Richtung auf den Basisabschnitt abgewinkelt ist (Anspruch4). Während sich die Federzungen zur Verrastung in der Siebträgerstruktur nach unten erstrecken, erstreckt sich der Stützschenkel entgegengesetzt zunächst schräg nach oben mit einem schräg nach oben erstreckten Schenkelabschnitt und dann wieder nach unten mit einem nach unten abgewinkelten Schenkelabschnitt zur Anlage an den Basisabschnitt des Instrumentenlagerteils. Hierdurch lässt sich eine nach unten, also in Richtung auf das Instrumentensieb, gerichtete Klemmkraft und insbesondere eine durch Federvorspannung erzeugte Klemmkraft auf den Basisabschnitt des Instrumentenlagerteils ausüben.

Es ist denkbar, dass der Wandabschnitt des Instrumentenlagerteils für die unmittelbare oder mittelbare Aufnahme der Instrumente und/oder Implantate ausgebildet ist (Anspruch 5). Er kann hierfür am oberen Ende etwa v-förmig erweitert sein oder zum Aufstecken oder Aufstülpen eines zusätzlichen Halters oder Formteils ausgebildet sein.

Es erweist sich weiter als vorteilhaft, wenn das Befestigungselement ein Blechbiegeteil ist und die Federzunge und der Stützschenkel aus dem Blechbiegeteil ausgeklinkt sind, also jeweils vom Material des Blechbiegeteils selbst gebildet sind (Anspruch 6).

Weiter wird vorgeschlagen, dass das Befestigungselement mehrere und einander vorzugsweise gegenüberliegend angeordnete, d.h. in einander gegenüberliegende Richtungen auslenkbare, Federzungen, insbesondere ein oder zwei Federzungen je Seite, aufweist und/oder mehrere und einander vorzugsweise gegenüberliegend angeordnete Stützschenkel aufweist (Anspruch 7). Es hat sich eine Ausführungsformen des Befestigungselements als bevorzugt erwiesen, welche zwei einander gegenüberliegend und in einander gegenüberliegende Richtungen auslenkbare Federzungen und zwei einander gegenüberliegend angeordnete Stützschenkel aufweist; ferner eine Ausführungsform, welche jeweils zwei Federzungen je Seite, also zwei mal zwei Federzungen jeweils einander gegenüberliegend, und wiederum zwei einander gegenüberliegende Stützschenkel aufweist.

Insbesondere bei der Ausbildung des Befestigungselements als Blechbiegeteil erweist es sich als vorteilhaft, wenn das Befestigungselement einen gegen die Oberseite der Siebträgerstruktur anlegbaren Grundschenkel und seitlich daran anschließend zwei einander gegenüberliegende Seitenschenkel aufweist (Anspruch 8).

Insbesondere erweist es sich als vorteilhaft, wenn die einander gegenüberliegenden Seitenschenkel des Befestigungselements eine nach oben offene U-Form begrenzen, die nach oben hin jedoch etwas verengt ist, indem die Seitenschenkel aufeinander zugeneigt sind und insbesondere am oberen Ende eine Abfalzung aufweisen können.

Es erweist sich als vorteilhaft, dass ausgehend von einem oder beiden Seitenschenkeln des Befestigungselements die jeweils wenigstens eine Federzunge ausgebildet ist und sich ausgehend von den Seitenschenkeln des Befestigungselements nach unten in Richtung auf die Siebträgerstruktur hin erstreckt (Anspruch 9).

Hierbei kann die Federzunge im Ausgangszustand des Befestigungselements derart vorkonfektioniert sein, dass sie mit dem Seitenschenkel einen spitzen Winkel einschließt und bezüglich des Seitenschenkels nach außen gebogen ist, insbesondere um 5° bis 25°.

Weiter erweist es sich als vorteilhaft, dass das Befestigungselement in die Ausnehmung in dem Basisabschnitt des Instrumentenlagerteils eintauchbar ist, so dass das Befestigungselement mit seinem Grundschenkel auf der Oberseite der Siebträgerstruktur positionierbar ist, und dass dann lediglich durch Ausüben einer Druckkraft auf das Befestigungselement in Richtung auf die Oberseite der Siebträgerstruktur die Federzunge ausgelenkt wird und dabei selbsttätig in eine Sieböffnung eindringt und dort mit einem die Sieböffnung begrenzenden Randbereich der Siebträgerstuktur verrastet, so dass das Befestigungselement und damit das Instrumentenlagerteil werkzeuglos und für den bestimmungsgemäßen Gebrauch unverlierbar an der Siebträgerstruktur befestigbar ist (Anspruch 10). Beim Niederdrücken oder Andrücken des in die Ausnehmung in dem Basisabschnitt eingesetzten Befestigungselements läuft die Federzunge oder die Federzungen also gegen einen eine Sieböffnung begrenzenden Randbereich der Siebträgerstrukur an; sie wird hierdurch bezüglich des Befestigungselements nach außen ausgelenkt und rastet dann in eine Hintergriffsituation, indem die Federzunge nach Eingreifen in eine Sieböffnung wieder zurückfedert und dabei einen die Sieböffnung begrenzenden Randbereich der Siebträgerstruktur hintergreift.

In Weiterbildung dieses Gedankens erweist es sich als vorteilhaft, dass ein freies Ende der Federzunge zur Bildung eines hintergreifenden Bereichs oder einer hintergreifenden Sicke ausgebildet oder umgeformt ist und dass ein freies Ende der Federzunge um mehr als 90°, vorzugsweise um wenigstens 100°, vorzugsweise um wenigstens 110° zur Längserstreckung der Federzunge abgewinkelt ist und somit einen Hintergriff mit einem eine Sieböffnung begrenzenden Randbereich der Siebträgerstruktur ausbildet (Anspruch 11).

Auch diesbezüglich erweist es sich als vorteilhaft, dass ein freies Ende der Federzunge eine Anlaufschräge bildet, welche beim Aufdrücken des Befestigungselements in Richtung auf die Oberseite der Siebträgerstruktur gegen einen eine Sieböffnung begrenzenden Randbereich der Siebträgerstruktur anläuft und dabei ausgelenkt wird, um in eine Hintergriffsstellung mit dem Randbereich zu rasten (Anspruch 12).

In Verbindung mit einer dreidimensional gewellten oder einer eierkartonähnlichen Erhebungen und Vertiefungen aufweisenden Siebträgerstruktur erweist es sich als vorteilhaft, wenn in dem Befestigungselement insbesondere in dem Grundschenkel des Befestigungselements, Aussparungen ausgebildet sind, in welche die Siebträgerstruktur mit einer Erhebung von unten eingreifen kann, so dass hierdurch eine weitere Lagestabilisierung des Befestigungselements an der Siebträgerstruktur erzielbar ist (Anspruch 13).

Gegenstand der Erfindung ist auch ein Instrumentensieb mit den Merkmalen des Anspruchs 14.

Weiter wird vorgeschlagen, dass das Instrumentensieb eine Siebträgerstruktur mit insbesondere rechteckförmigen und vorzugsweise quadratischen Sieböffnungen aufweist, die von gegenüber der Seitenlänge der Rechteckform oder gegenüber einer größten Abmessung der Sieböffnungen schmäleren Stegen begrenzt sind.

Es erweist sich als vorteilhaft, dass das Instrumentensieb in einer Draufsicht auf die Oberseite rechteckförmig ausgebildet ist mit einer dementsprechenden Längsrichtung und Querrichtung und dass die die Sieböffnungen begrenzenden Stege in einer Neigung von 45° zur Längsrichtung bzw. Querrichtung verlaufen.

Weiter erweist es sich als vorteilhaft, dass das Instrumentensieb eine 3-dimensional strukturierte, insbesondere gewellte Siebträgerstruktur umfasst, insbesondere, dass das Befestigungselement im Bereich von Hochpunkten, Erhebungen oder Bergen der Siebträgerstruktur anordenbar und befestigbar ist (Anspruch 15).

Es erweist sich auch als vorteilhaft, dass ein Abstand zweier benachbarter Federzungen des Befestigungselements einem Abstand zweier Kreuzungspunkte von die Sieböffnungen begrenzenden Stegen der Siebträgerstruktur entspricht oder das Doppelte oder ein ganzzahliges Vielfaches hiervon beträgt, so dass die Federzungen automatisch in jeweiligen Eckbereichen der Sieböffnungen positionierbar sind.

Bei einem erfindungsgemäßen Instrumentensieb mit einer erfindungsgemäßen Haltevorrichtung für Instrumente erweist es sich als vorteilhaft, wenn ein Löseinstrument vorgesehen ist, welches von innen gegen eine oder mehrere Federzungen des Befestigungselements anlegbar ist und hierdurch diese Federzunge aus ihrer Hintergriffstellung auslenkbar ist, so dass das Befestigungselement aus seiner Hintergriffsstellung bei dem Instrumentensieb gelöst werden kann.

Das Instrumentensieb kann vorteilhafterweise als Instrumentensiebkorb oder als ein Einsatz für einen Instrumentensiebkorb oder Instrumentenbehälter ausgebildet sein.

Weitere Merkmale, Einzelheiten und Vorteile der Erfindung ergeben sich aus den beigefügten Patentansprüchen aus der zeichnerischen Darstellung und nachfolgenden Beschreibung bevorzugter Ausführungsformen der Erfindung. In der Zeichnung zeigt:
- Figur 1: eine perspektivische explosionsartige Darstellung eines erfindungsgemäßen Instrumentensiebs mit einer Siebträgerstruktur und mit zwei erfindungsgemäßen Vorrichtungen zum Lagern eines nicht dargestellten Instruments, jeweils umfassend ein Instrumentenlagerteil und ein Befestigungselement zum lösbaren Festlegen des Instrumentenlagerteils an dem Instrumentensieb;
- Figur 2: eine perspektivische Darstellung der in Figur 1 dargestellten Instrumentenlagerteile;
- Figur 3a: eine perspektivische Darstellung einer ersten Ausführungsform des in Figur 1 dargestellten Befestigungselements zum lösbaren Festlegen des Instrumentenlagerteils an dem Instrumentensieb;
- Figur 3b: eine perspektivische Darstellung einer zweiten Ausführungsform des in Figur 1 dargestellten Befestigungselements zum lösbaren Festlegen des Instrumentenlagerteils an dem Instrumentensieb;
- Figur 4a: eine Draufsicht auf einen ebenen Basisabschnitt des Instrumentenlagerteils mit einer Ausnehmung zur Aufnahme des Befestigungselements nach Figur 3a;
- Figur 4b: eine Draufsicht auf einen ebenen Basisabschnitt des Instrumentenlagerteils mit einer Ausnehmung zur Aufnahme des Befestigungselements nach Figur 3b;
- Figur 5a eine Fig 4a: entsprechende Darstellung mit in die Ausnehmung eingesetztem Befestigungselement nach Fig 3a;
- Figur 5b eine Fig 4b: entsprechende Darstellung mit in die Ausnehmung eingesetztem Befestigungselement nach Fig 3b;
- Figuren 6a-f: verschiedene Ansichten zur Verdeutlichung der lösbaren Fixierung des Befestigungselements an der Siebträgerstruktur, und zwar zur leichteren Nachvollziehbarkeit ohne Zwischenordnung eines Instrumentenlagerteils und ohne Ausbildung des Befestigungselements mit einem erfindungsgemäßen Stützschenkel;
- Figur 7: eine Schnittansicht durch eine Siebträgerstruktur mit einer daran lösbar verrasteten in Figur 1 links dargestellten erfindungsgemäßen Vorrichtung zum Lagern eines nicht dargestellten Instruments, (welche Vorrichtung das in Figur 1 links dargestellte Instrumentenlagerteil und das in Figur 3b dargestellte Befestigungselement aufweist); und
- Figur 8: eine perspektivische Darstellung eines Löseinstruments für das Befestigungselement.

Die Figuren zeigen ein erfindungsgemäßes Instrumentensieb 2, für ärztliche, insbesondere chirurgische, Instrumente und/oder Implantate, zur Verwendung bei der Sterilgutversorgung im medizinischen Bereich. Das Instrumentensieb 2 umfasst eine Siebträgerstruktur 4 mit einer Vielzahl von Sieböffnungen 6 und schwebend oberhalb dargestellt beispielhaft zwei erfindungsgemäße Vorrichtungen 10 zum Lagern und vorzugsweise zum Halten eines in den Figuren nicht dargestellten Instruments während des Sterilgutkreislaufs. Jede Vorrichtung 10 umfasst hierbei ein Instrumentenlagerteil 12 mit je einem gegen die Siebträgerstruktur 4 anlegbaren Basisabschnitt 14 und zwei sich von dem Basisabschnitt 14 emporerhebenden Wandabschnitten 16 für die Aufnahme der nicht dargestellten Instrumente und/oder Implantate. Ferner umfasst jede Vorrichtung 10 ein Befestigungselement 18 zum lösbaren Festlegen des Instrumentenlagerteils 12 an dem Instrumentensieb 2 bzw. der Siebträgerstruktur 4, was nachfolgend noch erläutert werden wird.

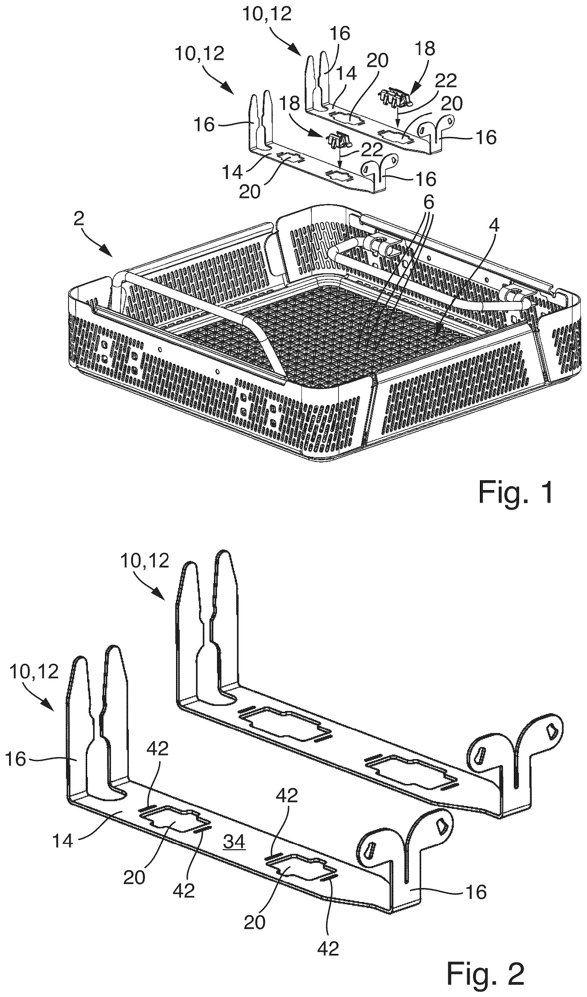

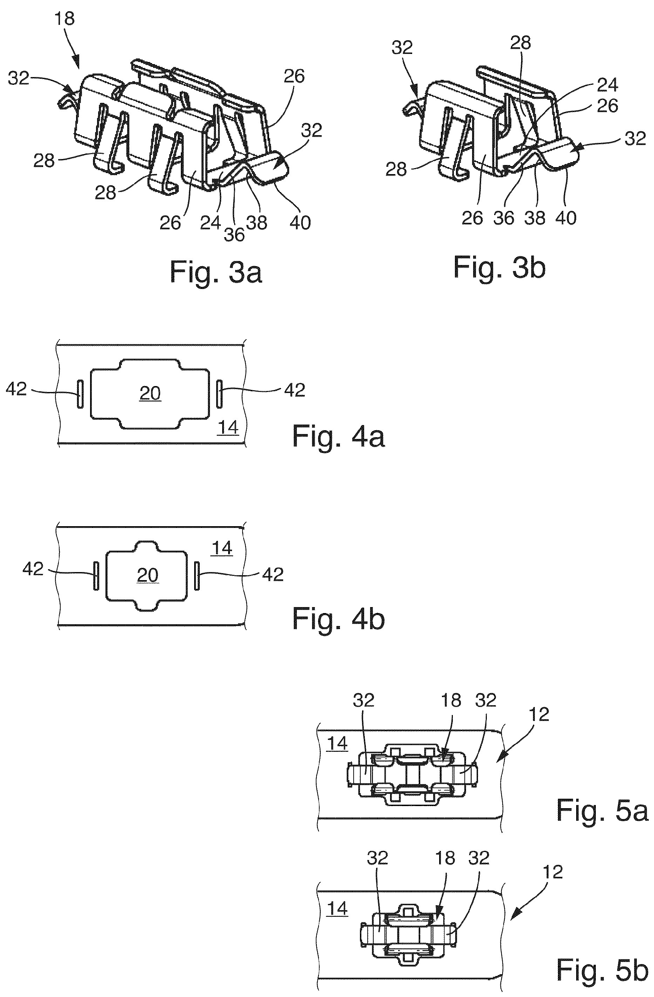

Die in Figuren 1 dargestellten Instrumentenlagerteile 12 und Befestigungselemente 16 sind vergrößert in Figur 2 und Figuren 3a, 3b dargestellt. Jedes Instrumentenlagerteil 12 umfasst einen im beispielhaft dargestellten Fall ebenen Basisabschnitt 14 und an den beiden Längsenden von dem Basisabschnitt 14 orthogonal nach oben vorstehende schon erwähnte Wandabschnitte 16 zur Aufnahme der Instrumenten. In dem Basisabschnitt 14 ist jeweils eine auf die Außenkontur des Befestigungselements 18 abgestimmte Ausnehmung 20 ausgebildet, so dass das Befestigungselement 18 in diese Ausnehmung 20 in Richtung des Pfeils 22 in Figur 1 einsteckbar ist und auf noch zu erläuternde Weise in eine formschlüssig wirkende Rastverbindung mit der Siebträgerstruktur 4 des Instrumentensiebs 2 gelangen kann. Hierbei übt das Befestigungselement 18 über beispielhaft zwei gegenüberliegende Stützschenkel 32 zugleich eine Klemmkraft auf den Basisabschnitt 14 des Instrumentenlagerteils 12 aus und fixiert damit zugleich das Instrumentenlagerteil 12 gegen die Siebträgerstruktur 4.

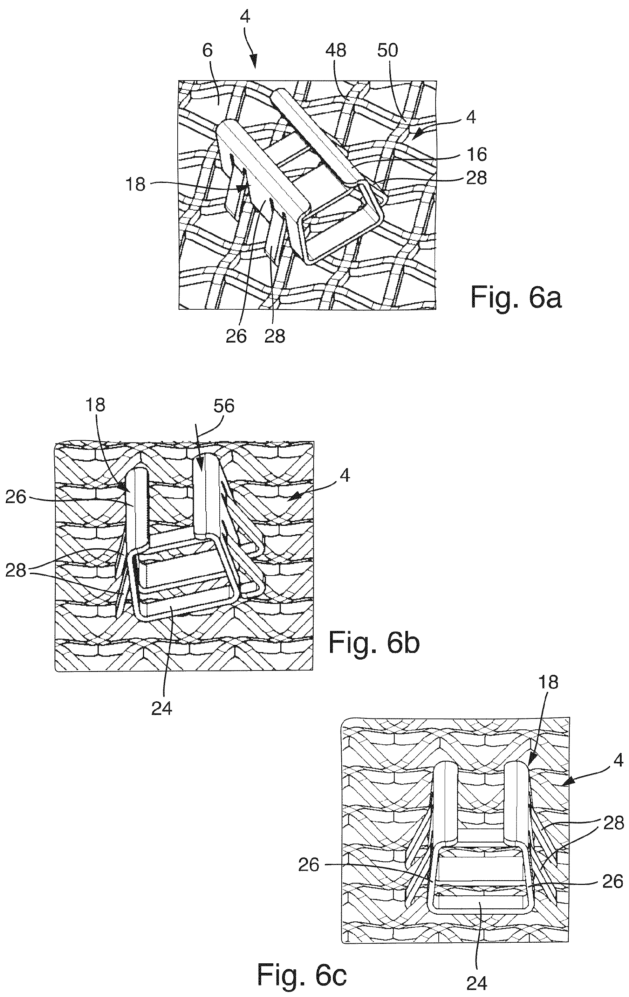

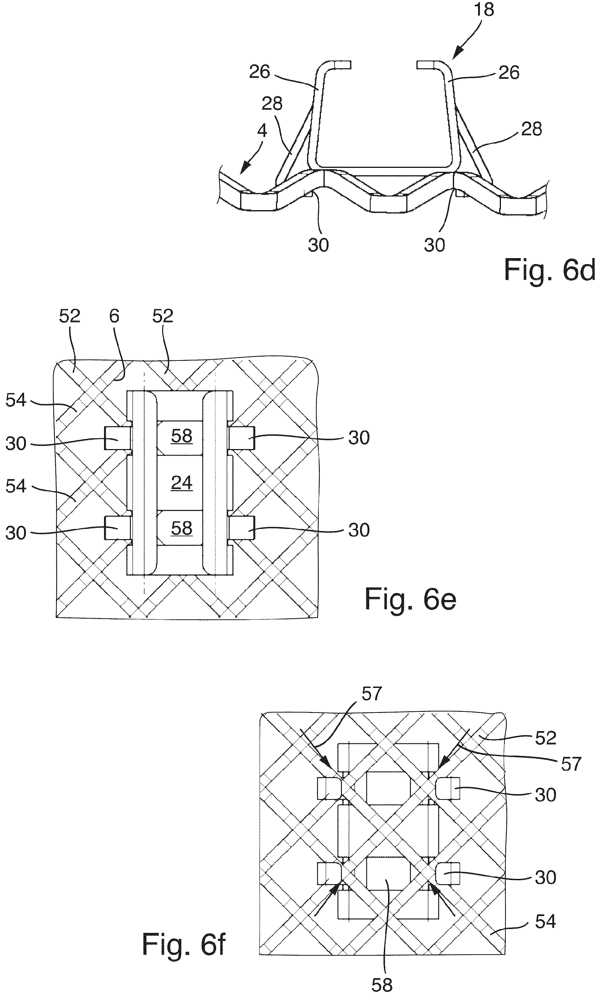

Das in Figuren 3a und 3b in zwei Ausführungsformen dargestellte Befestigungselement 18 ist beispielhaft von einem Blechbiegeteil gebildet. Es umfasst bei der beispielhaften Ausführungsform einen Grundschenkel 24 und seitlich daran anschießend zwei einander gegenüberliegende Seitenschenkel 26, so dass das Befestigungselement 18 eine ungefähre U-Form begrenzt. Ausgehend von einem oberen Bereich der Seitenschenkel 26 sind nach Figur 3a je Seitenschenkel jeweils zwei Federzungen 28 ausgebildet, die sich von oben nach unten, also in Richtung auf die Siebträgerstruktur 4, erstrecken. Sie haben an ihrem freien Ende einen abgewinkelten hintergreifenden Bereich 30, so dass sie mit der Siebträgerstruktur 4 und deren Sieböffnungen 6 verrastbar sind und so das Befestigungselement 18 unter Zwischenordnung des Basisabschnitts 14 des Instrumentenlagerteils 12 an der Siebträgerstruktur 4 zu halten und zu fixieren vermögen, was nachfolgend im einzelnen anhand der Figuren 6a-f noch erläutert wird. Des Weiteren weist das jeweilige Befestigungselement 18 im beispielhaft dargestellten Fall auf einander gegenüberliegenden Längsseiten je einen Stützschenkel 32 auf, mit dem sich das Befestigungselement 18 dann gegen eine von der Siebträgerstruktur 4 abgewandte obere Seite 34 des Basisabschnitts 14 des Instrumentenlagerteils 12 abstützt. Der jeweilige Stützschenkel 32 ist federnd ausgebildet und vermag so zusammen mit Instrumentenlagerteilen 12 mit unterschiedlich dicken Basisabschnitten 14 verwendet zu werden.

Der jeweilige Stützschenkel 32 ist ausgehend von einem unteren, also in die Ausnehmung 20 eingreifenden Bereich des Befestigungselements 18 ausgehend schräg nach oben erstreckt und ist dann wieder nach unten abgewinkelt. Der Stützschenkel 32 umfasst also einen von dem Grundschenkel 24 der U-Form schräg nach oben erstreckten Schenkelabschnitt 36 und daran anschließend einen nach unten abgewinkelten Schenkelabschnitt 38. Mit einem freien Ende 40 dieses nach unten abgewinkelten Schenkelabschnitts 38 drückt der Stützschenkel 32 auf den Basisabschnitt 14 des Instrumentenlagerteils 12 und übt die erwähnte Klemmkraft auf den Basisabschnitt 14 des Instrumentenlagerteils 12 aus. Hierdurch kann das Instrumentenlagerteil 12 gegen die Siebträgerstruktur 4 lösbar fixiert werden.

Zusätzlich zu der jeweiligen Ausnehmung 20 sind in dem Basisabschnitt 14 weitere im beispielhaften Fall schlitzförmige Ausnehmungen oder Vertiefungen 42 vorgesehen (s. Figur 1 und Figuren 4a, 4b), die eine Positionierhilfe für die Stützschenkel 32 bzw. deren freie Enden 40 bilden.

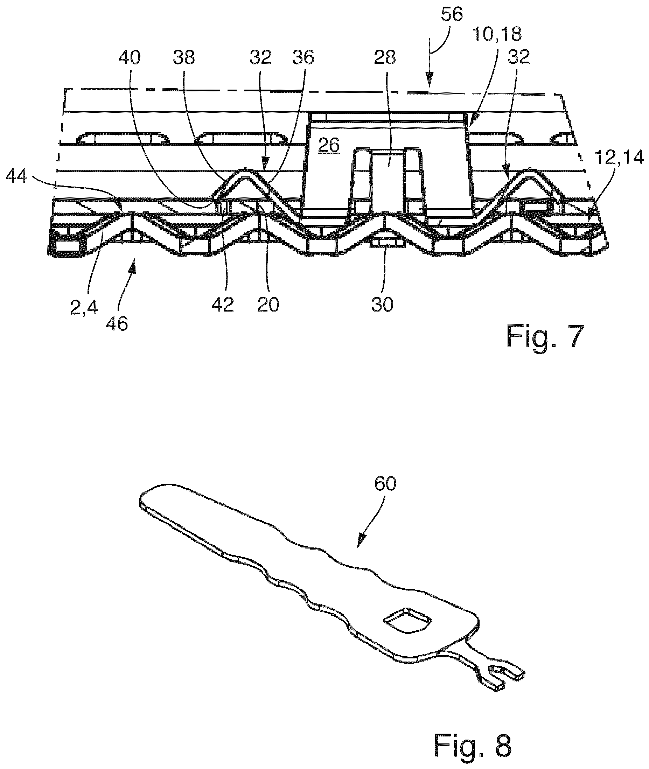

Das Befestigungselement 18 ist auf nachfolgend zu beschreibende Weise lösbar an der Siebträgerstruktur 4 befestigbar. Zur übersichtlicheren und verständlicheren Darstellung der Befestigung ist in den Figuren 6a-f das zwischen Befestigungselement 18 und Siebträgerstruktur 4 angeordnete Instrumentenlagerteil 12 jedoch nicht dargestellt sondern weggelassen. Ebenso ist der erfindungsgemäße Stützschenkel 32 weggelassen. Eine Schnittdarstellung mit dem auf erfindungsgemäße Weise zwischengeordneten Instrumentenlagerteil 12 mit Stützschenkel 32 ist in Figur 7 dargestellt. Die Siebträgerstruktur 4 in Figuren 6a-f weist eine der Vorrichtung 10 und den nicht dargestellten Instrumenten zugewandte Oberseite 44 und eine hiervon abgewandte Unterseite 46 auf. Im vorliegend dargestellten beispielhaften Fall ist die Siebträgerstruktur 4 in zwei Richtungen gewellt ausgebildet und weist so Erhebungen 48 und Vertiefungen 50 auf. Die Siebträgerstruktur 4 ist beispielhaft gebildet aus zwei Gruppen aus jeweils einer Vielzahl von projiziert auf die Ebene (siehe Fig 6e,f) parallel zueinander verlaufenden Stegen 52, 54, wobei die Stege 52 der einen Gruppe rechtwinklig zu den Stegen 54 der anderen Gruppe verlaufen, so dass sie die Sieböffnungen 6 begrenzen. Ausgehend von einer zunächst ebenen Siebträgerstruktur 4 aus den Stegen 52, 54 wurde dann die Struktur mit Erhebungen 48 und Vertiefungen 50 so aufgeprägt, dass in Richtung der Stege 52, 54 Erhebungen 48 und Vertiefungen 50 abwechseln. Trotz der dreidimensionalen Struktur können die Sieböffnungen 6 vorliegend beispielhaft als im Wesentlichen quadratisch bezeichnet werden, d.h. sie weisen bei senkrechter Projektion auf die Grundfläche der Siebträgerstruktur 4 eine quadratische Form auf (siehe Fig 6e, f).

Die Figuren 6a-f zeigen die beispielhaft dreidimensional gewellte Siebträgerstruktur 4 und das Befestigungselement 18 mit seinen Federzungen 28. Man erkennt in Figur 6a, b, dass das Befestigungselement 18 zunächst leicht gegen eine Ebene der Siebträgerstruktur 4 geneigt ist, so dass die in Figuren 6a, b links dargestellten Federzungen 28 des Seitenschenkels 26 im Bereich je einer Erhebung 48 oder Wellenberg der Siebträgerstruktur 4 in zwei benachbarte Sieböffnungen 6 eingreifen und mit ihrem jeweiligen hintergreifenden Bereich 30 mit den Stegen 52, 54 im Bereich eines Kreuzungspunkts verhaken. Ausgehend von dieser Montagesituation braucht nun lediglich eine Druckkraft in Richtung eines in Figur 6b dargestellten Pfeils 56 auf das Befestigungselement 18 bzw. den anderen Seitenschenkel 26 in Richtung auf die Siebträgerstruktur 4 ausgeübt zu werden, damit die gegenüberliegenden Federzungen 28 mit ihren hintergreifenden Endbereichen 30 gegen die Stege 53, 54 anlaufen und hierbei nach außen ausgelenkt werden und schließlich in die in Figur 6c-f dargestellte Hintergriffsituation gelangen, in der die Federzungen 28 nach Eingreifen in zwei Sieböffnungen 6 wieder nach innen zurückfedern und hierdurch eine unverlierbare Montageposition an der Siebträgerstruktur 4 einnehmen. Die hintergreifenden Endbereiche 30 der Federzungen 28 gelangen selbstzentrierend in Richtung der Pfeile 57 in Fig 6f gegen die Kreuzungspunkte der Stege 52, 54 der Sieböffnungen 6. In dieser Montageposition ruht das Befestigungselement 18 gewissermaßen mit seinem Grundschenkel 20 im Bereich von Erhebungen 48 der Siebträgerstruktur 4 und ist wieder parallel zur Ebene der Siebträgerstruktur 4 angeordnet. Das Befestigungselement 18 ruht aber bei der beispielhaft dargestellten Ausführungsform nicht ganz oben auf den Hochpunkten der Erhebungen 48 der Siebträgerstruktur 4, sondern die Hochpunkte oder Gipfel der Erhebungen 48 tauchen von unten etwas in Aussparungen 58 im Bereich des Grundschenkels 24 des Befestigungselements 18 ein, die sich hier durch das Ausklinken der Federzungen 28 ergeben haben.

Figur 7 zeigt in einer Schnittdarstellung die lösbare Montage der erfindungsgemäßen Vorrichtung 10 nunmehr mit Instrumentenlagerteil 12 und Befestigungselement 18 an der gewellten Siebträgerstruktur 4. Wie eingangs beschrieben wird das Instrumentenlagerteil 12 mit seinem Basisabschnitt 14 auf die Siebträgerstruktur 4 aufgelegt. Sodann wird ein jeweiliges Befestigungselement 18 in die jeweilige Ausnehmung 20 von oben eingetaucht, so dass es mit seinem Grundschenkel 24 auf der gewellten Siebträgerstruktur 4 aufliegt. Das Befestigungselement 18 wird dann wie vorausgehend (ohne Zwischenordnung des Instrumentenlagerteils 12 anhand der Figuren 6a-f) beschrieben mit der Siebträgerstruktur 4 lösbar verrastet. Man erkennt auch in Figur 7, dass der jeweilige hintergreifende Bereich 30 am freien Ende der jeweiligen Federzunge 28 die Siebträgerstruktur 4 im Bereich einer Erhebung 48 untergreift und dort im Kreuzungsbereich von Stegen 52, 54 verhakt ist, nachdem das Befestigungselement 18 in Richtung des Pfeils 56 gegen die Siebträgerstruktur 4 gedrückt wurde. In diesem Zustand werden die beiden gegenüberliegenden Stützschenkel 32 etwas nach oben belastet und üben so über den nach unten abgewinkelten Schenkel 38 eine Klemmkraft auf den Basisabschnitt 14 des Instrumentenlagerteils 12 aus. Sie greifen mit ihren freien Enden 40 in die jeweilige schlitzförmige Ausnehmung 42 in dem Basisabschnitt 14 ein und sind hierdurch positioniert. Durch die über die Stützschenkel 32 auf den Basisabschnitt 14 eingeleitete Klemmkraft wird das Instrumentenlagerteil 12 unverdrehbar und verliersicher an der Siebträgerstruktur 4 lösbar fixiert.

Zum Lösen der Haltevorrichtung 10 von der Siebträgerstruktur 4 kann mittels eines in Figur 8 dargestellten Löseinstruments 60 Zugriff auf die Federzungen 28 genommen werden, um die Federzungen 28 entgegen ihrer Federspannung nach außen auszulenken, so dass sie aus ihrer Hintergriffsituation mit den Stegen 52, 54 der Siebträgerstruktur 4 freikommen und das Befestigungselement 18 von der Siebträgerstruktur 4 zunächst nach oben abgeschwenkt und dann zur anderen Seite bewegt werden kann, damit auch die Federzungen 28 am gegenüberliegenden Seitenschenkel 26 aus ihrer Hintergriffsituation freikommen und das Befestigungselement 18 dann nach oben von der Siebträgerstruktur 4 und aus der Ausnehmung 20 im Basisabschnitt 14 des Instrumentenlagerteils 12 abgenommen werden kann.

Alternativ wäre es bei entsprechender Ausbildung der Länge und Vorspannung der Federzungen 26 aber grundsätzlich auch denkbar, dass das Befestigungselement 18 werkzeuglos aus der Hintergriffstellung mit den Stegen 52, 54 freikommen und dann nach oben abgenommen werden kann.

## Patentansprüche

1. Vorrichtung (10) zum Lagern und vorzugsweise zum Halten eines oder mehrerer ärztlicher, insbesondere chirurgischer, Instrumente und/oder Implantate in einer bestimmungsgemäßen Anordnung auf einem Instrumentensieb (2) zur Verwendung bei der Sterilgutversorgung im medizinischen Bereich mit einer Siebträgerstruktur (4) mit einer Vielzahl von Sieböffnungen (6), wobei die Vorrichtung (10) ein, insbesondere metallisches Instrumentenlagerteil (12) mit einem gegen die Siebträgerstruktur (4) anlegbaren Basisabschnitt (14) und mit wenigstens einem sich von dem Basisabschnitt (14) emporerhebenden Wandabschnitt (16) für die Aufnahme der Instrumente und/oder Implantate und ein Befestigungselement (18) zum lösbaren Festlegen des Instrumentenlagerteils (12) an der Siebträgerstruktur (4) des Instrumentensiebs (2) umfasst,
**dadurch gekennzeichnet,**
**dass** das Befestigungselement (18) wenigstens eine in Richtung auf die Siebträgerstruktur (4) des Instrumentensiebs (2) sich erstreckende und damit verrastbare Federzunge (28) und wenigstens einen gegen den Basisabschnitt (14) des Instrumentenlagerteils (12) abstützbaren Stützschenkel (32) aufweist,
und **dass** in dem Basisabschnitt (14) des Instrumentenlagerteils (12) eine auf die Außenkontur des Befestigungselements (18) derart abgestimmte und durch den Basisabschnitt (14) hindurchgehende Ausnehmung (20) ausgebildet ist, dass das Befestigungselement (18) mitsamt seiner Federzunge (28) in die Ausnehmung (20) eintauchbar ist, so dass die Federzunge (28) des Befestigungselements (18) mit der Siebträgerstruktur (4) verrastbar ist und sich das Befestigungselement (18) dann mit seinem Stützschenkel (32) gegen eine von der Siebträgerstruktur (4) abgewandte Seite (34) des Basisabschnitts (14) des Instrumentenlagerteils (12) abstützt, so dass das Instrumentenlagerteil (12) hierdurch an dem Instrumentensieb (2) fixiert ist.

2. Vorrichtung (10) nach Anspruch 1, **dadurch gekennzeichnet, dass** der Stützschenkel (32) des Befestigungselements (18) federnd ausgebildet ist, so dass verschiedene Dicken des Basisabschnitts (14) des Instrumentenlagerteils (12) hierdurch spielfrei akkommodiert werden können.

3. Vorrichtung (10) nach Anspruch 1 oder 2, **dadurch gekennzeichnet, dass** der Ausnehmung (20) in dem Basisabschnitt (14) des Instrumentenlagerteils (12) eine weitere insbesondere schlitzförmige Ausnehmung (42) oder Vertiefung zugeordnet und in dem Basisabschnitt (14) ausgebildet ist, in welche der Stützschenkel (32) des Befestigungselements (18) mit seinem freien Ende (40) eingreifen kann.

4. Vorrichtung (10) nach einem oder mehreren der vorstehenden Ansprüche, **dadurch gekennzeichnet, dass** sich der Stützschenkel (32) ausgehend von einem in die Ausnehmung (20) in dem Basisabschnitt (14) des Instrumentenlagerteils (12) eintauchbaren dem Instrumentensieb (2) zugewandten unteren Bereich des Befestigungselements durch diese Ausnehmung schräg nach oben über den Basisabschnitt (14) hinaus erstreckt und dann wieder in Richtung auf den Basisabschnitt abgewinkelt ist.

5. Vorrichtung (10) nach einem oder mehreren der vorstehenden Ansprüche, **dadurch gekennzeichnet, dass** der Wandabschnitt (16) des Instrumentenlagerteils (12) für eine unmittelbare oder mittelbare Aufnahme der Instrumente und/oder Implantate ausgebildet ist.

6. Vorrichtung (10) nach einem oder mehreren der vorstehenden Ansprüche, **dadurch gekennzeichnet, dass** das Befestigungselement (18) ein Blechbiegeteil ist und die Federzunge (28) und der Stützschenkel (32) aus dem Blechbiegeteil ausgeklinkt sind.

7. Vorrichtung (10) nach einem oder mehreren der vorstehenden Ansprüche, **dadurch gekennzeichnet, dass** das Befestigungselement (18) mehrere und einander vorzugsweise gegenüberliegend, d.h. in einander gegenüberliegende Richtungen auslenkbare, Federzungen (28), insbesondere ein oder zwei Federzungen (28) je Seite, aufweist und/oder mehrere und einander vorzugsweise gegenüberliegend angeordnete Stützschenkel (32) aufweist.

8. Vorrichtung (10) nach einem oder mehreren der vorstehenden Ansprüche, **dadurch gekennzeichnet, dass** das Befestigungselement (18) einen gegen die Siebträgerstruktur (4) anlegbaren Grundschenkel (24) und seitlich daran anschließend zwei einander gegenüberliegende Seitenschenkel (26) aufweist.

9. Vorrichtung (10) nach Anspruch 8, **dadurch gekennzeichnet, dass** ausgehend von einem oder beiden Seitenschenkeln (26) des Befestigungselements (18) die jeweils wenigstens eine Federzunge (28) ausgebildet ist und sich ausgehend von den Seitenschenkeln (26) des Befestigungselements (18) nach unten in Richtung auf die Siebträgerstruktur (4) hin erstreckt, insbesondere mit dem Seitenschenkel (26) einen spitzen Winkel einschließt und bezüglich des Seitenschenkels (26) nach außen gebogen ist, insbesondere um 5° - 25°.

10. Vorrichtung (10) nach einem der Ansprüche 8 oder 9, **dadurch gekennzeichnet, dass** das Befestigungselement (18) in die Ausnehmung (20) in dem Basisabschnitt (14) des Instrumentenlagerteils (12) eintauchbar ist, so dass das Befestigungselement (18) mit seinem Grundschenkel (24) auf der Oberseite (44) der Siebträgerstruktur (4) positionierbar ist, und dass dann lediglich durch Ausüben einer Druckkraft auf das Befestigungselement (18) in Richtung auf die Oberseite (44) der Siebträgerstruktur (4) die Federzunge (28) ausgelenkt wird und dabei selbsttätig in eine Sieböffnung (6) eindringt und dort mit einem die Sieböffnung (6) begrenzenden Randbereich der Siebträgerstuktur (4) verrastet, so dass das Befestigungselement (18) und damit das Instrumentenlagerteil (12) werkzeuglos und für den bestimmungsgemäßen Gebrauch unverlierbar an der Siebträgerstruktur (4) befestigbar ist.

11. Vorrichtung (10) nach einem oder mehreren der vorstehenden Ansprüche, **dadurch gekennzeichnet, dass** ein freies Ende der Federzunge (28) zur Bildung eines hintergreifenden Bereichs (30) oder einer hintergreifenden Sicke ausgebildet oder umgeformt ist und dass das freie Ende der Federzunge (28) vorzugsweise um mehr als 90°, vorzugsweise um wenigstens 100°, vorzugsweise um wenigstens 110° zur Längserstreckung der Federzunge (28) abgewinkelt ist und somit einen Hintergriff mit einem eine Sieböffnung (6) begrenzenden Randbereich der Siebträgerstruktur (4) ausbildet.

12. Vorrichtung (10) nach einem oder mehreren der vorstehenden Ansprüche, **dadurch gekennzeichnet, dass** ein freies Ende der Federzunge (28) eine Anlaufschräge bildet, welche beim Aufdrücken des Befestigungselements (18) in Richtung auf die Oberseite (44) der Siebträgerstruktur (4) gegen einen eine Sieböffnung (6) begrenzenden Randbereich der Siebträgerstruktur (4) anläuft und dabei ausgelenkt wird, um in eine Hintergriffsstellung mit dem Randbereich zu rasten.

13. Vorrichtung (10) nach einem oder mehreren der vorstehenden Ansprüche, **dadurch gekennzeichnet, dass** in dem Befestigungselement (18), insbesondere in dem Grundschenkel (24) des Befestigungselements (18), Aussparungen (58) ausgebildet sind, in welche die Siebträgerstruktur (4) mit einer Erhebung (48) von unten eingreifen kann, so dass hierdurch eine weitere Lagestabilisierung des Befestigungselements (18) an der Siebträgerstruktur (4) erzielbar ist.

14. Instrumentensieb (10) für ärztliche, insbesondere chirurgische Instrumente und/oder Implantate zur Verwendung bei der Sterilgutversorgung im medizinischen Bereich, insbesondere OP-Bereich, mit einer Siebträgerstruktur (4) mit einer Vielzahl von Sieböffnungen (6), **gekennzeichnet durch** eine Vorrichtung (10) nach einem oder mehreren der vorstehenden Ansprüche.

15. Instrumentensieb (10) nach Anspruch 14, **dadurch gekennzeichnet, dass** das Instrumentensieb (2) eine 3-dimensional strukturierte, insbesondere gewellte Siebträgerstruktur (4) umfasst, insbesondere dass das Befestigungselement (18) im Bereich von Hochpunkten, Erhebungen (48) oder Bergen der Siebträgerstruktur (4) anordenbar und befestigbar ist.
